# EUROPEAN PATENT APPLICATION

(11) **EP 1 994 942 A1**
(43) Date of publication of application: **26.11.2008**
(21) Application number: 07301064.7
(22) Date of filing: 25.05.2007
(51) Int. Cl.: A61K 38/45, C07K 7/08, C12N 15/00, C12N 5/00

(54) **Pharmaceutical compositions comprising telomerase, and uses thereof**

(71) Applicant: INSERM (Institut National de la Santé et de la Recherche Médicale), 75654 Paris Cedex 13 (FR)
(72) Inventor: Kellermann, Guillaume, 75014 Paris (FR)
(74) Representative: Chajmowicz, Marion

(57) **Abstract**

The present invention provides a pharmaceutical composition comprising a therapeutically effective amount of human telomerase reverse transcriptase (hTERT) protein or, a hTERT dominant negative variant thereof, or a function-conservative homolog thereof comprising the protein transduction domain (PTD) of hTERT, wherein the protein does not comprise and is not conjugated to any heterologous internalisation moiety.

## Description

The present invention relates to pharmaceutical compositions comprising human telomerase reverse transcriptase (hTERT) protein or variants thereof, and uses thereof.

### Background of the invention:

Telomerase is a ribonucleoprotein that catalyzes the addition of telomeric repeats to the ends of telomeres. Telomeres are long stretches of repeated sequences that cap the ends of chromosomes and are believed to stabilize the chromosome. In humans, telomeres are typically 7-10 kb in length and comprise multiple repeats of the sequence- TTAGGG-.

Because most malignant cells rely on the activity of the protein telomerase for their immortality, it has been proposed that a drug which inactivates telomerase might be effective against a broad spectrum of malignancies. It has been demonstrated that telomerase inhibition quickly led cancer cells to apoptosis (Hahn et al., 1999). At the same time, most healthy tissues in the body express a low level of telomerase, and would function normally in its absence. Adult human beings with no telomerase activity could be identified (Xin et al., 2006).

Telomerase was proposed as a potential effective target for anti-tumour drugs (see International Patent Applications WO 93/23572, WO 99/65875, WO 01/02377, WO 01/02394, WO 01/80855, WO 02/051409, WO 02/053155, WO 02/076397, and WO 2005/023994). Inhibitors of telomerase activity include chemical molecules (e.g. thiazolidinediones or oxime derivatives) and biological molecules (e.g. substituted flavone compounds or modified oligonucleotides).

International Patent Application WO 98/14593 discloses of the interest of using human telomerase reverse transcriptase (hTERT) for treating various diseases, including cancers. Two clinical trials involving telomerase inhibitors have been conducted by Geron Corporation. One uses a therapeutic cancer vaccine comprised of autologous cells loaded ex vivo with hTERT mRNA (GRNVAC1). The other uses a lipidated oligonucleotide drug (GRN163L), the lipid moiety enhancing cellular uptake which increases efficacy and makes it possible to reduce dosage.

Furthermore, telomerase expression plays a role in cellular senescence, as it is normally repressed in postnatal somatic cells resulting in progressive shortening of telomeres. Cellular senescence is the phenomenon where cells lose the ability to divide. The successive shortening of the chromosomal telomeres with each cell cycle is believed to limit the number of divisions of the cell, thus contributing to ageing. Hayflick observed that cells dividing in cell culture divided about 50 times before entering an irreversible growth-arrest state (Shay et al., 2000). This limit is believed to be one of the causes of ageing. It is also believed that if the shortening of telomeres could be slowed down or prevented, life expectancy could be extended. Moreover, the shortening of telomeres is considered as a key factor in the occurrence of many ageing-related pathologies such as liver cirrhosis, ulcers, immunosenescence, atherosclerosis, type II diabetes, cataracts, osteoporosis and many other degenerative processes (Chang et al., 2004 and Marciniak et al. 2001). At last, it has been shown that telomerase activation at a middle level is insufficient for inducing tumors but sufficient for enhancing cellular regeneration potential (Hamad et al., 2002).

International Patent Application WO 2005/000245 discloses describes methods for inducing telomerase activity in cells in order to treat degenerative diseases.

Indeed, it is known that replication of a linear DNA strand by conventional DNA polymerases requires an RNA primer and can proceed only 5' to 3'. When the RNA bound at the extreme 5' ends of eukaryotic chromosomal DNA strands is removed, a gap is introduced leading to a progressive shortening of daughter strands with each round of replication. This shortening of telomeres, the protein-DNA structures physically located on the ends of chromosomes, is thought to account for the phenomenon of cellular senescence or aging of normal human somatic cells *in vitro* and *in vivo.* The length and integrity of telomeres thus impacts entry of a cell into a senescent stage (i.e., loss of proliferative capacity).

In order to exert its biological effect, telomerase or its splice variants must be delivered into the cell. However, there is a permanent bias in the art according to which a protein which is considered as a large molecule cannot cross cell membranes (Wadia et al., 2002). It is indeed generally known that substances with a molecular weight above 600 Daltons can hardly pass through cell membranes. hTERT has a calculated molecular weight of about 127 kiloDaltons.

Different transduction technologies have been applied to hTERT in order to ensure transduction into cells. International Patent Application WO00/61617 discloses the use of a fusion polypeptide comprising a protein transduction domain (PTD) such as those of TAT or VP22 and a second polypeptide having telomerase-specific activity such as hTERT to transiently immortalize a cell. A strategy based on the fusion of hTERT with a PTD has also been presented as a possibility to overcome replicative senescence during ex vivo culture of primary explanted cells (Heng et al., 2005) or to treat liver cirrhosis by injecting TAT-hTERT fusion directly in vivo (Maser et al., 2002).

Biotechnology companies propose different techniques to ensure transduction of hTERT into cells. For instance, Phoenix Biomolecular develops a Cell Penetrating Peptide (CPP)-based macromolecular delivery technology in order to transduce hTERT into a cell. Telomolecular Corp. proposes special biodegradable nanoparticles able to deliver large molecules, including telomerase, through cell membranes.

There is a continuing need in the art for delivering large molecules, such as telomerase, into a cell.

### Brief summary of the invention:

It has now been shown that hTERT may penetrate into a cell without any penetration-enhancing carrier. In particular no fusion to a penetration-enhancing peptide is needed.

Accordingly, the present invention provides a pharmaceutical composition comprising a therapeutically effective amount of human telomerase reverse transcriptase (hTERT) protein or, an hTERT dominant negative variant thereof, or a function-conservative homolog thereof comprising the protein transduction domain (PTD) of hTERT, wherein the protein does not comprise and is not conjugated to any heterologous internalisation moiety. Most advantageously, the protein is not conjugated to any peptide or vehicle enhancing penetration of the protein into cells.

In a preferred embodiment, the pharmaceutical composition is free of any further internalisation carrier.

Another subject of the invention is the use of human telomerase reverse transcriptase (hTERT) protein, or a function-conservative homolog thereof comprising the protein transduction domain (PTD) of hTERT, for the manufacture of a medicament intended for increasing the proliferation capacity of a human cell and/or for preventing or treating a condition associated with a low level of telomerase activity in a human subject, wherein the protein does not comprise and is not conjugated to any heterologous internalisation moiety. In a particular embodiment, the condition involves cellular senescence.

A further subject of the invention is the use of a dominant negative variant of hTERT, preferably splice variant alpha of human telomerase reverse transcriptase (hTERTalpha) protein, or a function-conservative homolog thereof comprising the protein transduction domain (PTD) of hTERT, in the manufacture of a medicament intended for decreasing the proliferation capacity of a human cell and/or preventing or treating a human condition associated with a high level of telomerase activity in a human subject, wherein the protein does not comprise and is not conjugated to any heterologous internalisation moiety. In a particular embodiment, the condition is a cancer or involves a cellular hyperproliferation.

The present invention further identifies the protein transduction domain (PDT) of hTERT consisting of the amino acid sequence RRRGGSASRSLPLPKRPRR (SEQ ID NO: 2).

This PTD fragment is useful to transduce various peptides or polypeptides of interest.

Accordingly, the invention provides a chimeric polypeptide comprising the protein transduction domain of hTERT consisting of the amino acid sequence as shown in SEQ ID NO:2, fused to a peptide or polypeptide of interest, it being understood that the chimeric polypeptide is neither hTERT nor one of its splice variants. The chimeric polypeptide can be employed in a pharmaceutical composition or in an in vitro method for transducing said peptide or polypeptide of interest.

At last the invention thus provides an *in vitro* method for transducing a peptide or polypeptide of interest into a cell, said method comprising contacting the cell with a chimeric polypeptide as defined above, in a sufficient time to allow the transduction of the chimeric polypeptide into the cell.

### Detailed description of the invention:

### Definitions:

The maintenance of telomeres is a function of a telomere-specific DNA polymerase known as telomerase, which is a ribonucleoprotein polymerase that maintains telomere ends by addition of the telomere repeat TTAGGG since in humans and other vertebrates telomeric DNA consists of hundreds to thousands of tandem repeats of the sequence TTAGGG.

More precisely, the enzyme consists of a protein component (hTERT) with reverse transcriptase activity, encoded by this gene, and an RNA component (hTR) which serves as a template for the telomere repeat.

Alternatively spliced variants encoding different isoforms of telomerase reverse transcriptase have been identified. Among these, a transcript variant, also called alpha, uses an in-frame alternate splice site in the coding region, compared to the wild-type isoform. This variant is shorter than the hTERT isoform and lacks part of reverse transcriptase (RT) motif. Alpha transcript variant is known to be a dominant-negative inhibitor of telomerase activity (Golgin et al, 2000).

The term "hTERT" is used herein to define the polypeptide sequence of human telomerase reverse transcriptase. The naturally occurring protein has an aminoacid sequence shown in Genbank, Accession number AAC51672.1 (SEQ ID NO: 1). The term "hTERT" designates the naturally occurring protein as well as function-conservative variants or homologues. The protein can show post-translational modifications such as glycosylation, or phosphorylation, or other modifications of some amino acid residues.

A dominant negative variant is a polypeptide which differs from the naturally occurring form of hTERT by at least one mutation in the active site required for biological activity of hTERT, i.e. the reverse transcriptase activity. A preferred dominant negative variant is the alpha splice variant (Colgin LM. et al. 2000 and Yi X. et al. 2000) (Genbank, Accession number NP 937986, SEQ ID NO: 3) or dominant negative point mutation variants (Hahn WC. et al. 1999 and Harrington L. et al. 1997).

The proteins used in the present invention can be isolated from nature or can be prepared using standard recombinant and/or synthetic methods. Variants or homologues include proteins that are substantially identical (e.g., that have 80, 85, 90, 95, 97, 98, 99%, sequence identity) to a native sequence. Such variants include proteins having amino acid alterations such as deletions, truncations, insertions and/or substitutions. A "deletion" refers to the absence of one or more amino acid residues in the related protein. The term "insertion" refers to the addition of one or more amino acids in the related protein. A "substitution" refers to the replacement of one or more amino acid residues by another amino acid residue in the polypeptide. Typically, such alterations are conservative in nature such that the activity of the variant protein is substantially similar to a native sequence protein (see, e.g., Creighton (1984) Proteins, W.H. Freeman and Company). In the case of substitutions, the amino acid replacing another amino acid usually has similar structural and/or chemical properties. Insertions and deletions are typically in the range of 1 to 5 amino acids, although depending upon the location of the insertion, more amino acids can be inserted or removed. The variations can be made using methods known in the art such as site-directed mutagenesis, cassette mutagenesis, restriction selection mutagenesis, and PCR mutagenesis.

"Function-conservative variants" are those in which a given amino acid residue in a protein or enzyme has been changed without altering the overall conformation and function of the polypeptide, including, but not limited to, replacement of an amino acid with one having similar properties (such as, for example, polarity, hydrogen bonding potential, acidic, basic, hydrophobic, aromatic, and the like). Amino acids other than those indicated as conserved may differ in a protein so that the percent protein or amino acid sequence similarity between any two proteins of similar function may vary and may be, for example, from 70 % to 99 % as determined according to an alignment scheme such as by the Cluster Method, wherein similarity is based on the MEGALIGN algorithm. A "function-conservative variant" also includes a polypeptide which has at least 60 % amino acid identity as determined by BLAST or FASTA algorithms, preferably at least 75 %, most preferably at least 85%, and even more preferably at least 90 %, and which has the same or substantially similar properties or functions as the native or parent protein to which it is compared.

The term "function-conservative", when applied to hTERT variant or homolog, means that the variant or homolog shows telomerase activity. The term "function-conservative", when applied to hTERT dominant negative variant or homolog, means that the variant or homolog shows an inhibitory activity on telomerase activity.

In the context of the present invention, the hTERT protein or variants thereof is characterized as being "free of any heterologous internalisation moiety". The term "heterologous internalisation moiety" means a component that promotes or facilitates the delivery or the entry into a cell of the protein of interest to which it is conjugated or linked. Internalisation moieties include protein transduction domain or cell permeable peptide, e.g., TAT, polyarginine, polylysine, polyamine, poly-ornithine, cationic peptides, low-molecular weight protamin, penetratin, homeodomain, helix-loop-helix region, DNA or RNA binding regions, heparin binding domain, transportan, Pep-1, Wr-T, PTD5, SynB1, Diatos peptide vector, Hph-1 PTD, NeuroD PTD, Prion PTD.

Preferably, the composition is free of any further internalisation carriers.

"Further internalisation carriers" include naturally entering proteins (e.g., protamin, histones, antibodies, FGF, PDCD5, Period1), viral components (e.g., Herpes VP22, hemagglutinating virus of Japan Envelope), protein transfection reagents (e.g., Chariot^{™}, Pro-Ject^{™}, TransPass^{™} P, ProteoJuice^{™}, PULSin^{™}), cationic lipids, liposomes, nanoparticles (e.g., poly(lactic-co-glycolic acid)), dendrimers, polycation or small-molecule carriers (e.g., Okuyama et al., 2007).

The hTERT protein can be in isolated form or may be associated to other proteins, peptides, nucleotides, lipids, or to any other chemical or biochemical components which do not behave as an intracellular transport vehicle, but can improve the stability, activity, or biodistribution.

### Therapeutic methods and uses:

It is herein described a method for preventing or treating a telomerase-related condition, which method comprises administering to a patient in need thereof a therapeutically effective amount of hTERT or hTERT dominant negative variant, wherein the protein does not comprise and is not conjugated to any heterologous internalisation moiety.

The patient may be any mammal, preferably a human being.

Administration of hTERT is particularly advantageous for treating a condition associated with a low level of telomerase activity,

The term "a condition associated with a low level of telomerase activity" is used herein to define conditions caused by an expression or activity of hTERT which is not sufficient enough to lengthen or maintain telomere length in a group of cells. The term "a low level of telomerase activity" thus means a level which is limiting to the replication potential. International Patent Application WO98/14593 cites such conditions, including cellular senescence related disorders, the effects of aging, infertility and others. Further examples of such conditions include (but are not limited to): Alzheimer's disease, stroke; age-related diseases of the integument such as dermal atrophy, elastolysis and skin wrinkling, sebaceous gland hyperplasia, senile lentigo, graying of hair and hair loss, chronic skin ulcers, and age-related impairment of wound healing; degenerative joint disease; osteoporosis: age-related immune system impairment (e.g., involving cells such as B and T lymphocytes, monocytes, neutrophils, eosinophils. basophils, NK cells and their respective progenitors): age-related diseases of the vascular system including atherosclerosis, calcification, thrombosis, and aneurysms, diabetes, muscle atrophy, respiratory diseases, diseases of the liver and Gl tract, metabolic diseases, endocrine diseases (e.g., disorders of the pituitary and adrenal gland), reproductive diseases, and age-related macular degeneration.

Administration of hTERT dominant negative variants is advantageous for treating a condition associated with a high level of telomerase activity.

The term "a condition associated with a high level of telomerase activity" is used herein to define conditions where hTERT lengthens or maintains telomeres in a group of cells. The term "associated with a high level of telomerase activity" thus means a situation where telomerase extends cellular replicative potential.

Cancers include solid tumors and leukemias. Types of cancer that may be treated include (but are not limited to): adenocarcinoma of the breast, prostate, and colon; all forms of bronchogenic carcinoma of the lung; myeloid, melanoma; hepatoma; neuroblastoma; papilloma; apudoma; choristoma; branchioma; malignant carcinoid syndrome; carcinoid heart disease; carcinoma (e.g., Walker, basal cell, basosquamous, Brown-Pearce, ductal, Ehrlich tumor, in situ, Krebs 2, Merkel cell, mucinous, non-small cell lung, oat cell, papillary, scirrhous, bronchiolar, bronchogenic, squamous cell, and transitional cell), histiocytic disorders; leukemia (e.g., B-cell, mixed-cell, null-cell, T-cell, T-cell chronic, HTLV-II-associated, lyphocytic acute, lymphocytic chronic, mast-cell, and myeloid); histiocytosis malignant; Hodgkin's disease; immunoproliferative small; non-Hodgkin's lymphoma; plasmacytoma; reticuloendotheliosis; melanoma; chondroblastoma; chondroma; chondrosarcoma; fibroma; fibrosarcoma; giant cell tumors; histiocytoma; lipoma; liposarcoma; mesothelioma; myxoma; myxosarcoma; osteoma; osteosarcoma; Ewing's sarcoma; synovioma; adenofibroma; adenolymphoma; carcinosarcoma; chordoma: craniopharyngioma; dysgerminoma; hamartoma; mesenchymoma; mesonephroma; myosarcoma; ameloblastoma; cementoma; odontoma; teratoma; thymoma; trophoblastic tumor; adenocarcinoma; adenoma; cholangioma; cholesteatoma; cylindroma; cystadenocarcinoma; cystadenoma; granulosa cell tumor; gynandroblastoma; hepatoma; hidradenoma; islet cell tumor; Leydig cell tumor; papilloma; Sertoli cell tumor; theca cell tumor; leiomyoma; leiomyosarcoma; myoblastoma; myoma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin; angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma (e.g., Ewing's, experimental, Kaposi's, and mast-cell); neoplasms (e.g., bone, breast, digestive systems colorectal, liver, pancreatic, pituitary, testicular, orbital, head and neck, central nervous system, acoustic, pelvic, respiratory tract, and urogenital); neurofibromatosis, and cervical dysplasia).

hTERT dominant negative variant may also be beneficial to prevent the development of T cell memory, and thus treat or prevent autoimmune diseases (e.g., multiple sclerosis, polyarthritis) or improve long term tolerance of organs after allotransplantation.

In the context of the invention, the term "treating" or "treatment", as used herein, means reversing, alleviating, inhibiting the progress of, or preventing the disorder or condition to which such term applies, or one or more symptoms of such disorder or condition.

By a "therapeutically effective amount" of hTERT, or hTERT dominant negative variant, as above described, is meant a sufficient amount of hTERT or hTERT dominant negative variant, both free of any heterologous internalisation moiety, to treat telomerase-related disease or disorder at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific polypeptide employed; and like factors well known in the medical arts. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 10,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the patient to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 50 mg/kg of body weight per day, especially from about 0.001 mg/kg to 7 mg/kg of body weight per day.

### Pharmaceutical compositions:

The invention provides pharmaceutical compositions comprising a therapeutically effective amount of an isolated or recombinant polypeptide having the amino acid sequence of hTERT protein, preferably having the aminoacid sequence shown as SEQ ID NO: 1, or its function-conservative variants, free of any heterologous internalisation moiety.

The invention also provides pharmaceutical compositions comprising a therapeutically effective amount of isolated or recombinant polypeptide corresponding to a hTERT dominant negative variant or its function-conservative variants, free of any heterologous internalisation moiety.

The invention further provides pharmaceutical compositions comprising a therapeutically effective amount of a chimeric polypeptide comprising the protein transduction domain of hTERT consisting of the amino acid sequence as shown in SEQ ID NO:2, fused to a peptide or polypeptide of interest.

hTERT, hTERT dominant negative variants or chimeric proteins of the present invention, all free of any heterologous internalisation moiety, may be combined with pharmaceutically acceptable excipients, and optionally sustained-release matrices, such as biodegradable polymers, to form therapeutic compositions.

The telomerase proteins (i.e. the hTERT protein, an hTERT dominant negative variant, or a function-conservative homolog thereof) can be administered alone or in combination with the telomerase RNA component (hTR) which serves as a template for the telomere repeat addition, or with a truncated or mutated form or hTR. The telomerase proteins, optionally complexed with hTR, may be further combined with other telomerase interacting factors, such as dyskerin, chaperones, heat shock protein 90, p23, or any other factors associated with hTERT or hTR.

In a particular embodiment, the telomerase proteins may be combined with any additional agent useful for preventing or treating a telomerase-related condition.

In particular, hTERT dominant negative variants free of any heterologous internalisation moiety may also be associated with another chemotherapy agent such as 5-fluorouracil, fluorodeoxyuridine, vinca alkaloids, cyclophosphamide and other nitrogen mustard alkylating agents, daunorubicin, doxorubicin, methotrexate, cytosine arabinoside, 6-mercaptopurine, thioguanosine, podophyllotoxins or cisplatin.

hTERT dominant negative variants can also be combined with anti-angiogenic agents, such as Bevacizumab (Avastin®).

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The pharmaceutical compositions of the present invention can be formulated for intravenous, local, subcutaneous, transdermal, oral, sublingual, intramuscular or rectal administration. The active principle (i.e. the telomerase proteins), alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms.

Preferably, the pharmaceutical compositions contain vehicles which are pharmaceutically acceptable for a formulation capable of being injected. Preferably the pharmaceutical composition is slowly perfused intravenously to reach organs or metastases inside the body, or is injected at the site of the tissue to be transduced, e.g. at the site of a tumor. hTERT and its variants which comprise the endogenous PTD should pass through the blood-brain barrier and the placenta barrier (Schwarze et al, 1999).

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi.

The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active polypeptides in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed.

For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The hTERT and related polypeptides may be formulated within a therapeutic mixture to comprise about 0.0001 to 1000 milligrams, or about 0.001 to 0.1 milligrams, or about 0.1 to 1.0 or even about 1000 milligrams per dose or so. Multiple doses can also be administered.

In addition to the polypeptides formulated for parenteral administration, such as intravenous or intramuscular injection, other pharmaceutically acceptable forms include, e.g. tablets or other solids for oral administration; time release capsules; and any other form currently used.

### Protein transduction domain:

The inventor has shown that a fragment of hTERT with the following sequence: RRRGGSASRSLPLPKRPRR (SEQ ID NO: 2) has the ability to act as a Protein transduction domain PTD (hTERT PTD).

The PTD polypeptide can be synthesized by any technique known in the art, e.g. by expression in a host cell.

A hTERT PTD-encoding polynucleotide can be inserted into an expression vector which contains the necessary elements for the transcription and translation of the inserted coding sequence. Transcription and translation control elements include, for example, a promoter (e.g., T7 or T3), ribosome binding site, start codon, stop codon, and polyadenylation site.

The transduction domain can form a covalent bond with a peptide, or polypeptide, so that the peptide or polypeptide is transduced into a cell without the need of a separate receptor or carrier, or energy. A fusion protein can be expressed from approximately in frame-fused gene-coding sequences in known suitable host cells. Corresponding polynucleotide sequences can be prepared and manipulated using elements of known and standard recombinant DNA techniques and readily available adaptations thereof.

A nucleotide sequence coding for a peptide, oligopeptide, polypeptide or protein of interest can thus be linked to hTERT PTD-encoding polynucleotide in order to form a covalent bond with the hTERT PTD and thus obtain a fusion protein showing biological activity when delivered into a cell.

Chemically-coupled products can be used if desired, and can be prepared from the individual protein components according to any of a variety of chemical coupling techniques known in the art.

The delivery of the peptide or polypeptide of interest into the cell is improved by the presence of the PTD fragment.

The term "peptide or polypeptide of interest" means a therapeutic, preventive or diagnostic peptide, or polypeptide, which penetration into a cell is rendered possible, or is enhanced, by the PTD of hTERT.

Non-limiting examples of peptide or protein of interest include enzymes, hormones, cytokines, antibodies or antibody fragments, peptide fragments (e.g. peptides recognised by antibodies).

The present invention also provides a method for transducing a polypeptide or peptide of interest into a cell by producing a fusion protein between hTERT PTD and peptide or polypeptide of interest. The fusion protein can be administered in vivo or can be useful in a method for transducing cells in vitro.

In the latter embodiment, the *in vitro* method for transducing a peptide or polypeptide of interest into a cell, comprises the steps of:
(a) producing a fusion protein as described above, and
(b) contacting the cell with said fusion protein in a sufficient time to allow the transduction of the fusion protein into the cell.

### Ex vivo applications

The present invention further provides an ex *vivo* method for culturing or amplifying cells, preferably progenitor or stem cells, which method comprises contacting cells with a hTERT protein or a function-conservative homolog thereof, said protein or homolog being free of any internalisation moiety; and culturing the cells under conditions allowing them to amplify.

The amplified cells are useful in cell therapy or for producing organs to graft. In a particular embodiment, the cell is a progenitor cell (e.g. fibroblasts, keratinocytes, corneal cells, retinal pigment epithelial cells, odontoblast, endothelial cells, smooth muscle cells, lymphocytes, hematopoietic stem cells, neural precursor cells, mesenchymal stem cells). After amplification, the progenitor cell can be grafted into a patient affected with degenerative disease such as age-related macular degeneration, immune senescence, AIDS, aplastic anaemia, Parkinson or Huntington's disease. The amplified progenitor cells may also be useful to fight skin aging.

The cells to amplify may further be primary cells which are useful in the research field. More generally, the hTERT protein or a function-conservative homolog thereof, would thus be useful in a culture medium, or in a kit to prepare a culture medium.

The invention will further be illustrated in view of the following figures and examples.

### Description of the figures:

Figure 1 shows a schematic representation of wild type catalytic subunit of telomerase (hTERT) and of a fusion gene between hTERT and HIV-TAT protein transduction domain (TAT-hTERT). (HA: Hemagglutinin Tag)
Figure 2 shows western blots of hTERT and TAT-hTERT recombinant proteins after partial purification from insect cells infected with recombinant baculovirus coding for hTERT or TAT-hTERT. Only TAT-hTERT fusion protein contains the HA-Tag, and could be detected using an anti-Ha antibody, whereas both TAT-hTERT and hTERT proteins could be detected using an anti-hTERT antibody Extracts from uninfected cells gave no signal.
Figure 3 shows a western blot of primary human fibroblasts incubated one hour with hTERT, or TAT-HA-hTERT, or PBS (Control). Before cell lysis, cultures where washed four times with PBS to remove excess of proteins. TAT-hTERT but surprisingly also native hTERT could be detected in cell extracts.
Figure 4 shows intensity of immunofluorescence performed with an anti-hTERT antibody on NIH-3T3 cells incubated with hTERT, or TAT-hTERT, or PBS (Control). Control experiment shows background signal of the full immunofluorescence on untreated cells.
Figure 5 shows intensity of intracellular fluorescence detected by confocal microscopy of HuH7 cells incubated with the control peptide (FITC-ARPLEHGSDKAT, SEQ ID NO: 4) or with the hTERT PTD peptide (FITC-RRRGGSASRSLPLPKRPRR). Whereas control peptide was excluded from cells, hTERT PTD concentrated into the nucleus of lived cells.
Figure 6 shows detection of telomerase activity, using the Telomerase Repeat Assay Protocol (TRAP), in primary human fibroblasts previously incubated with hTERT protein.

### EXAMPLES:

### Materials and methods

### Materials:

**Cell culture:** NIH3T3 cells were cultured in Dulbecco's Modified Eagle's Medium (DMEM) supplemented with 10% new born calf serum. HuH7 and Human primary fibroblasts (Biochimie, Hôpital Bicêtre) were cultured in DMEM/10% fetal calf serum.

**Generation of recombinant Baculovirus:** hTERT cDNA (provided by Robert Weinberg, MIT) was used as a template to PCR amplify hTERT N-Terminal coding sequence, using primers 5'TAATTACCATGGGAATGCCGCGCGCTCC 3' (SEQ ID NO: 5) and 5' TCGGTCCACGCGTCCTGC 3'.(SEQ ID NO: 6) PCR products were purified, digested with Ncol/Mlul. HTERT cDNA was digested by Mlul/Sall and both fragments were inserted into Ncol/Xhol sites of pTAT-HA vector (provided by Steven Dowdy, HHMI) to create a His-TAT-HA-TERT fusion gene. His-TAT-HA-hTERT fragment (XbaI/EcoRI) was inserted into (BamH1/Ecorl) pFastBac 1 vector using the BamH1-Xbal adaptors: 5' GATCCAGAGT 3' (SEQ ID NO 7) and 5' CTAGACTCTG 3'.(SEQ ID NO 8) Wild type hTERT fragment (Ecorl/Sall) was inserted into (Ecorl/Sall) pFastBac 1 vector (Invitrogen). Recombinant baculovirus were generated using Bac-to-Bac® system (Invitrogen), according to supplier instructions.

Baculovirus were amplified on sf21 cells (Invitrogen) cultured in TC-100 medium (Sigma) supplemented with 10% fetal calf serum. H5 cells (Invitrogen) were used for virus titration and protein expression. 90% confluent H5 cells, cultured into Express Five® SFM, were infected with recombinant baculovirus at a multiplicity of infection of 3 and incubated for 48 hours at 27°C to allow recombinant protein expression.

**Protein isolation from insect cells:** Infected cells were harvested, washed in phosphate-buffered saline (PBS), and resuspended in one volume of 20mM Tris pH 8,0 supplemented with protease inhibitor cocktail (complete, Roche). Cells were lysed by sonication (three pulses of ten seconds). The non-soluble fraction was removed by centrifugation 5 minutes at 16000 g. Extract was incubated 30 minutes in cold room with equal volume of DEAE sepharose (Sigma-Aldrich). The non-bound fraction containing partially purified hTERT or His-TAT-HA-hTERT was recovered after centrifugation 5 minutes at 16000 g.

**Protein transduction:** Partially purified hTERT or His-TAT-HA-hTERT proteins were mixed with an equal volume of DMEM, and applied for one hour to cell cultures.

**Western Blot analysis:** Western blots were performed according to standard methods and were detected with the enhanced chemiluminescence system (ECL, Amersham). Samples were resolved on 7% SDS-PAGE. His-TAT-HA-TERT and hTERT were detected with an anti-hTERT antibody (1:1000, clone Y182 Epitomics), followed by anti-rabbit secondary antibody (Amersham, NA934V). His-TAT-HA-TERT was also detected with an anti-HA antibody (1:1000, Covance) followed by antimouse secondary antibody (Amersham, NA931V).

**Immunofluorescence:** After three washes in PBS, cells were fixed (4% formaldehyde), permeabilized with 1 % Triton X-100 and saturated with 1% Bovine Serum Albumin. Cells were incubated with the anti-hTERT antibody (1:100, clone Y182 Epitomics), washed in PBS and incubated with Alexa Fluor® 488 chicken anti-rabbit IgG (1:800, Invitrogen A21441). Coverslips were observed with flurorescence microscopy (Leika HBO100) and images were captured by using a digital camera (Nikon DXM1200F).

**Telomerase activity:** Telomerase activity was evaluated using the telomeric repeat assay protocol according to supplier instructions (TRAPeze® Telomerase Detection Kit, Qbiogene).

**Peptide transduction:** Control peptide ARPLEHGSDKAT (Mi et al., Mol Ther 2000) and hTERT putative protein transduction domain RRRGGSASRSLPLPKRPRR were synthesized and linked to FITC (Fluorescein Iso Thio Cyanate) at the N Terminus (Eurogenetec). Peptides were resuspended in DMEM and were applied to HuH7 (human hepatoma) cultures at 50µM for one hour. Cells were washed five times with DMEM without red phenol and lived cells were observed by confocal microscopy.

**Quantification:** Fluorescence quantifications were performed using Image-J software.

### RESULTS:

### Transduction of hTERT into a cell:

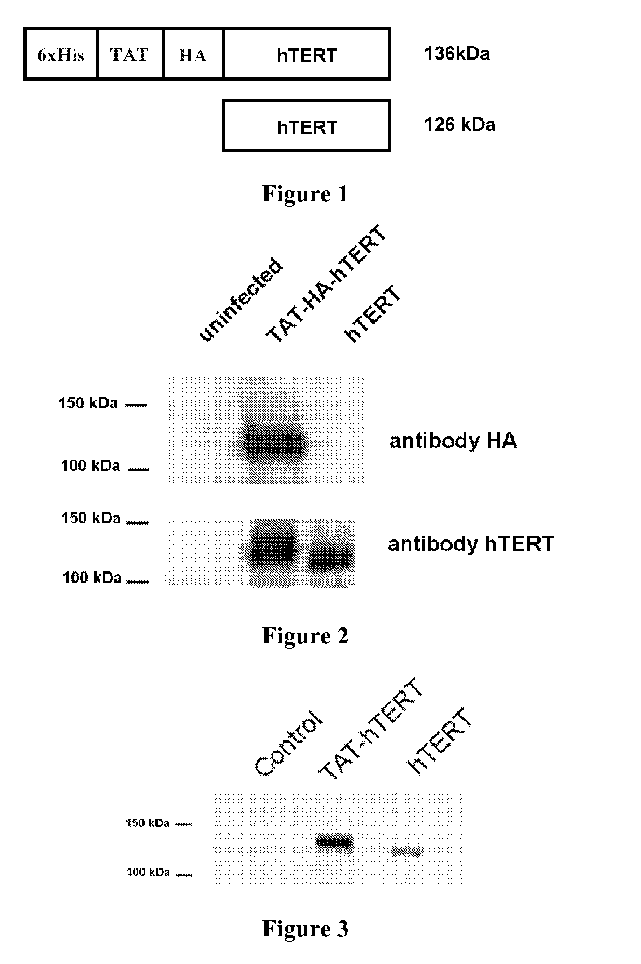

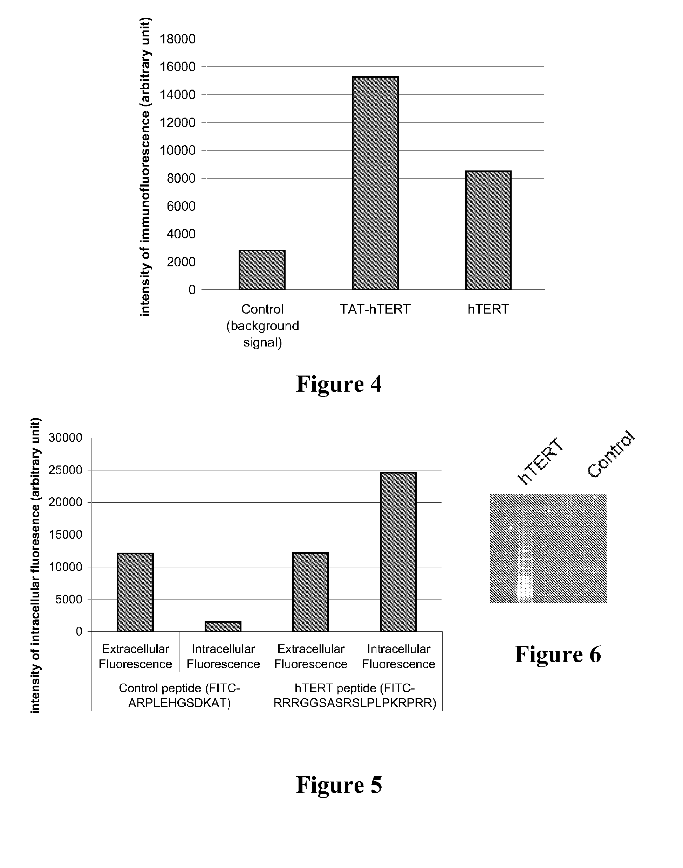

hTERT was shown to transduce into cells without any additional transduction agent. Figure 3 shows hTERT internalization into primary human fibroblasts. hTERT internalisation was also analysed by immunofluorescence (Figure 4).

Comparatively with TAT-hTERT internalization, the signal intensity is only slightly lower for native hTERT (factor 3 between hTERT and TAT-hTERT internalization). These results are unusual, as most proteins (B-galactosidase, p53...) are undetectable on western blot after transduction, because only very few proteins can normally enter. Efficient detection requires fusion to a PTD like HIV-TAT which then dramatically enhances the internalisation capabilities (factor above 100 (Mi et al., 2000)). The easy detection of native hTERT and the very low enhancement of its transfer by TAT PTD both suggest that hTERT already owns a functional PTD inside its sequence.

In conclusion, these experiments show that native hTERT penetrates into a cell without the help of any penetration-enhancer.

### Identification of hTERT protein transduction domain:

Protein transduction domains (PTDs) are often basic peptides found in nucleic acid binding regions (Futaki et al., 2001). As hTERT contains a DNA as well as a RNA binding region, hTERT sequence was analyzed for a putative PTD. Inside the RNA binding region, a possible candidate long of 19 amino acids was identified. In vitro, this sequence proved to be able to deliver a fluorescent molecule (FITC) into human cells, whereas a control peptide was inefficient, as shown on figure 5. These data demonstrate that hTERT contains a PTD, which had not been previously identified.

### Activity of internalized hTERT:

Internalized hTERT has been demonstrated to be active by a Telomerase Repeat Assay Protocol (TRAP) as shown on figure 6. As activity measurement was performed six hours after protein transduction, these data suggest that hTERT remains biologically active after the transduction process.

### REFERENCES:

Chang S, Multani AS, Cabrera NG, Naylor ML, Laud P, Lombard D, Pathak S, Guarente L, DePinho RA. Essential role of limiting telomeres in the pathogenesis of Werner syndrome. Nat Genet. 2004 Aug;36(8):877-82.
Colgin LM, Wilkinson C, Englezou A, Kilian A, Robinson MO, Reddel RR. The hTERT alpha splice variant is a dominant negative inhibitor of telomerase activity. Neoplasia. 2000 Sep-Oct; 2(5):426-32.
Futaki S, Suzuki T, Ohashi W, Yagami T, Tanaka S, Ueda K, Sugiura Y. Arginine-rich peptides. An abundant source of membrane-permeable peptides having potential as carriers for intracellular protein delivery. J Biol Chem. 2001 Feb 23;276(8):5836-40.
Hahn WC, Stewart SA, Brooks MW, York SG, Eaton E, Kurachi A, Beijersbergen RL, Knoll JH, Meyerson M, Weinberg RA. Inhibition of telomerase limits the growth of human cancer cells. Nat Med. 1999 Oct; 5(10):1164-70.
Hamad NM, Banik SS, Counter CM. Mutational analysis defines a minimum level of telomerase activity required for tumourigenic growth of human cells. Oncogene. 2002 Oct 10;21(46):7121-5
Harrington L, Zhou W, McPhail T, Oulton R, Yeung DS, Mar V, Bass MB, Robinson MO. Human telomerase contains evolutionarily conserved catalytic and structural subunits. Genes Dev. 1997 Dec 1; 11(23):3109-15.
Heng BC, Cao T. Making cell-permeable recombinant telomerase (transtelomerase) through fusion of its catalytic subunit (hTERT) with protein transduction domains (PTD): a possible strategy to overcome replicative senescence during ex vivo culture of primary explanted cells. Med Hypotheses. 2005; 65(1):199-200.
Jemal A, Murray T, Ward E, Samuels A, Tiwari RC, Ghafoor A, Feuer EJ, Thun MJ. Cancer statistics, 2005. CA Cancer J Clin. 2005 Jan-Feb; 55(1):10-30.
Jemal A, Siegel R, Ward E, Murray T, Xu J, Smigal C, Thun MJ. Cancer statistics, 2006. CA Cancer J Clin. 2006 Mar-Apr;56(2):106-30.
Marciniak R, Guarente L. Human genetics. Testing telomerase.Nature. 2001 Sep 27;413(6854):370-1, 373
Maser RS, DePinho RA. Connecting chromosomes, crisis, and cancer. Science. 2002 Jul 26;297(5581):565-9. Review.
Mi Z, Mai J, Lu X, Robbins PD. Characterization of a class of cationic peptides able to facilitate efficient protein transduction in vitro and in vivo. Mol Ther. 2000 Oct;2(4):339-47.
Okuyama M, Laman H, Kingsbury SR, Visintin C, Leo E, Eward KL, Stoeber K, Boshoff C, Williams GH, Selwood DL. Small-molecule mimics of an alpha-helix for efficient transport of proteins into cells. Nat Methods. 2007 Feb;4(2):153-9.
Schwarze SR, Ho A, Vocero-Akbani A, Dowdy SF. In vivo protein transduction: delivery of a biologically active protein into the mouse. Science. 1999 Sep 3;285(5433):1569-72.
Shay JW, Wright WE. Hayflick, his limit, and cellular ageing. Nat Rev Mol Cell Biol. 2000 Oct; 1(1):72-6.
Wadia JS, Dowdy SF. Protein transduction technology. Curr Opin Biotechnol. 2002 Feb;13(1):52-6. Review.
Xin ZT, Beauchamp AD, Calado RT, Bradford JW, Regal JA, Shenoy A, Liang Y, Lansdorp PM, Young NS, Ly H. Functional characterization of natural telomerase mutations found in patients with hematologic disorders. Blood. 2007 Jan 15;109(2):524-32.
Yi X, White DM, Aisner DL, Baur JA, Wright WE, Shay JW. An alternate splicing variant of the human telomerase catalytic subunit inhibits telomerase activity. Neoplasia. 2000 Sep-Oct; 2(5):433-40.

## Claims

1. A pharmaceutical composition comprising human telomerase reverse transcriptase (hTERT) protein, a hTERT dominant negative variant, or a function-conservative homolog thereof comprising the protein transduction domain (PTD) of hTERT, wherein the protein does not comprise and is not conjugated to any heterologous internalisation moiety.

2. The pharmaceutical composition of claim 1, wherein the dominant negative variant is human telomerase reverse transcriptase splice variant alpha (hTERTalpha)

3. The pharmaceutical composition of any of claims 1 or 2, further comprising hTR RNA.

4. The pharmaceutical composition of any of claims 1 to 3, which is free of any further internalisation carrier.

5. Use of human telomerase reverse transcriptase (hTERT) protein or a function-conservative homolog thereof comprising the protein transduction domain (PTD) of hTERT, for the manufacture of a medicament intended for increasing the proliferation capacity of a human cell and/or for preventing or treating a condition associated with a low level of telomerase activity in a human subject, wherein the protein does not comprise and is not conjugated to any heterologous internalisation moiety.

6. The use according to claim 5, wherein the condition involves cellular senescence.

7. Use of a dominant negative variant of human telomerase reverse transcriptase protein, or a function-conservative homolog thereof comprising the protein transduction domain (PTD) of hTERT, in the manufacture of a medicament intended for decreasing the proliferation capacity of a human cell and/or preventing or treating a condition associated with an high level of telomerase activity in a human subject, wherein the protein does not comprise and is not conjugated to any heterologous internalisation moiety.

8. The use of claim 7, wherein the dominant negative variant is human telomerase reverse transcriptase splice variant alpha (hTERTalpha).

9. The use according to claim 7 or 8, wherein the condition is a cancer or involves a cellular hyperproliferation.

10. The use according to claim 7 or 8, wherein the medicament is intended for preventing or treating a auto-immune disease.

11. The use according to claim 7, wherein the medicament is intended for improving long term tolerance of organs after allotransplantation.

12. Protein transduction domain of hTERT consisting of the amino acid sequence RRRGGSASRSLPLPKRPRR (SEQ ID NO: 2).

13. A chimeric polypeptide comprising the protein transduction domain of hTERT consisting of the amino acid sequence as shown in SEQ ID NO:2, fused to a peptide or polypeptide of interest, it being understood that the chimeric polypeptide is neither hTERT nor one of its splice variants.

14. A pharmaceutical composition comprising the chimeric polypeptide of claim 13.

15. An *in vitro* method for transducing a peptide or polypeptide of interest into a cell, said method comprising contacting the cell with a chimeric polypeptide as defined in claim 13, in a sufficient time to allow the transduction of the chimeric polypeptide into the cell.

16. An ex *vivo* method for culturing or amplifying cells, which method comprises contacting cells with a hTERT protein or a function-conservative homolog thereof, and culturing the cells under conditions allowing them to amplify, wherein the protein does not comprise and is not conjugated to any heterologous internalisation moiety.


SEQUENCE LISTING

<110>  INSERM

<120>  Pharmaceutical compositions comprising telomerase, and uses thereof

<130>  BO585EP

<160>  8

<170>  PatentIn version 3.3

<210>  1
<211>  1132
<212>  PRT
<213>  Homo sapiens

<400>  1

<210>  2
<211>  19
<212>  PRT
<213>  homo sapiens

<400>  2

Arg Arg Arg Gly Gly Ser Ala Ser Arg Ser Leu Pro Leu Pro Lys Arg
1               5                   10                  15

Pro Arg Arg

<210>  3
<211>  1120
<212>  PRT
<213>  homo sapiens

<400>  3

Met Pro Arg Ala Pro Arg Cys Arg Ala Val Arg Ser Leu Leu Arg Ser
1               5                   10                  15

His Tyr Arg Glu Val Leu Pro Leu Ala Thr Phe Val Arg Arg Leu Gly
            20                  25                  30

Pro Gln Gly Trp Arg Leu Val Gln Arg Gly Asp Pro Ala Ala Phe Arg
        35                  40                  45

Ala Leu Val Ala Gln Cys Leu Val Cys Val Pro Trp Asp Ala Arg Pro
    50                  55                  60

Pro Pro Ala Ala Pro Ser Phe Arg Gln Val Ser Cys Leu Lys Glu Leu
65                  70                  75                  80

Val Ala Arg Val Leu Gln Arg Leu Cys Glu Arg Gly Ala Lys Asn Val
                85                  90                  95

Leu Ala Phe Gly Phe Ala Leu Leu Asp Gly Ala Arg Gly Gly Pro Pro
            100                 105                 110

Glu Ala Phe Thr Thr Ser Val Arg Ser Tyr Leu Pro Asn Thr Val Thr
        115                 120                 125

Asp Ala Leu Arg Gly Ser Gly Ala Trp Gly Leu Leu Leu Arg Arg Val
    130                 135                 140

Gly Asp Asp Val Leu Val His Leu Leu Ala Arg Cys Ala Leu Phe Val

145                 150                 155                 160

Leu Val Ala Pro Ser Cys Ala Tyr Gln Val Cys Gly Pro Pro Leu Tyr
                165                 170                 175

Gln Leu Gly Ala Ala Thr Gln Ala Arg Pro Pro Pro His Ala Ser Gly
            180                 185                 190

Pro Arg Arg Arg Leu Gly Cys Glu Arg Ala Trp Asn His Ser Val Arg
        195                 200                 205

Glu Ala Gly Val Pro Leu Gly Leu Pro Ala Pro Gly Ala Arg Arg Arg
    210                 215                 220

Gly Gly Ser Ala Ser Arg Ser Leu Pro Leu Pro Lys Arg Pro Arg Arg
225                 230                 235                 240

Gly Ala Ala Pro Glu Pro Glu Arg Thr Pro Val Gly Gln Gly Ser Trp
                245                 250                 255

Ala His Pro Gly Arg Thr Arg Gly Pro Ser Asp Arg Gly Phe Cys Val
            260                 265                 270

Val Ser Pro Ala Arg Pro Ala Glu Glu Ala Thr Ser Leu Glu Gly Ala
        275                 280                 285

Leu Ser Gly Thr Arg His Ser His Pro Ser Val Gly Arg Gln His His
    290                 295                 300

Ala Gly Pro Pro Ser Thr Ser Arg Pro Pro Arg Pro Trp Asp Thr Pro
305                 310                 315                 320

Cys Pro Pro Val Tyr Ala Glu Thr Lys His Phe Leu Tyr Ser Ser Gly
                325                 330                 335

Asp Lys Glu Gln Leu Arg Pro Ser Phe Leu Leu Ser Ser Leu Arg Pro
            340                 345                 350

Ser Leu Thr Gly Ala Arg Arg Leu Val Glu Thr Ile Phe Leu Gly Ser
        355                 360                 365

Arg Pro Trp Met Pro Gly Thr Pro Arg Arg Leu Pro Arg Leu Pro Gln
    370                 375                 380

Arg Tyr Trp Gln Met Arg Pro Leu Phe Leu Glu Leu Leu Gly Asn His
385                 390                 395                 400

Ala Gln Cys Pro Tyr Gly Val Leu Leu Lys Thr His Cys Pro Leu Arg
                405                 410                 415

Ala Ala Val Thr Pro Ala Ala Gly Val Cys Ala Arg Glu Lys Pro Gln
            420                 425                 430

Gly Ser Val Ala Ala Pro Glu Glu Glu Asp Thr Asp Pro Arg Arg Leu
        435                 440                 445

Val Gln Leu Leu Arg Gln His Ser Ser Pro Trp Gln Val Tyr Gly Phe
    450                 455                 460

Val Arg Ala Cys Leu Arg Arg Leu Val Pro Pro Gly Leu Trp Gly Ser
465                 470                 475                 480

Arg His Asn Glu Arg Arg Phe Leu Arg Asn Thr Lys Lys Phe Ile Ser
                485                 490                 495

Leu Gly Lys His Ala Lys Leu Ser Leu Gln Glu Leu Thr Trp Lys Met
            500                 505                 510

Ser Val Arg Asp Cys Ala Trp Leu Arg Arg Ser Pro Gly Val Gly Cys
        515                 520                 525

Val Pro Ala Ala Glu His Arg Leu Arg Glu Glu Ile Leu Ala Lys Phe
    530                 535                 540

Leu His Trp Leu Met Ser Val Tyr Val Val Glu Leu Leu Arg Ser Phe
545                 550                 555                 560

Phe Tyr Val Thr Glu Thr Thr Phe Gln Lys Asn Arg Leu Phe Phe Tyr
                565                 570                 575

Arg Lys Ser Val Trp Ser Lys Leu Gln Ser Ile Gly Ile Arg Gln His
            580                 585                 590

Leu Lys Arg Val Gln Leu Arg Glu Leu Ser Glu Ala Glu Val Arg Gln
        595                 600                 605

His Arg Glu Ala Arg Pro Ala Leu Leu Thr Ser Arg Leu Arg Phe Ile
    610                 615                 620

Pro Lys Pro Asp Gly Leu Arg Pro Ile Val Asn Met Asp Tyr Val Val
625                 630                 635                 640

Gly Ala Arg Thr Phe Arg Arg Glu Lys Arg Ala Glu Arg Leu Thr Ser
                645                 650                 655

Arg Val Lys Ala Leu Phe Ser Val Leu Asn Tyr Glu Arg Ala Arg Arg
            660                 665                 670

Pro Gly Leu Leu Gly Ala Ser Val Leu Gly Leu Asp Asp Ile His Arg
        675                 680                 685

Ala Trp Arg Thr Phe Val Leu Arg Val Arg Ala Gln Asp Pro Pro Pro
    690                 695                 700

Glu Leu Tyr Phe Val Lys Asp Arg Leu Thr Glu Val Ile Ala Ser Ile
705                 710                 715                 720

Ile Lys Pro Gln Asn Thr Tyr Cys Val Arg Arg Tyr Ala Val Val Gln
                725                 730                 735

Lys Ala Ala His Gly His Val Arg Lys Ala Phe Lys Ser His Val Ser
            740                 745                 750

Thr Leu Thr Asp Leu Gln Pro Tyr Met Arg Gln Phe Val Ala His Leu
        755                 760                 765

Gln Glu Thr Ser Pro Leu Arg Asp Ala Val Val Ile Glu Gln Ser Ser
    770                 775                 780

Ser Leu Asn Glu Ala Ser Ser Gly Leu Phe Asp Val Phe Leu Arg Phe
785                 790                 795                 800

Met Cys His His Ala Val Arg Ile Arg Gly Lys Ser Tyr Val Gln Cys
                805                 810                 815

Gln Gly Ile Pro Gln Gly Ser Ile Leu Ser Thr Leu Leu Cys Ser Leu
            820                 825                 830

Cys Tyr Gly Asp Met Glu Asn Lys Leu Phe Ala Gly Ile Arg Arg Asp
        835                 840                 845

Gly Leu Leu Leu Arg Leu Val Asp Asp Phe Leu Leu Val Thr Pro His
    850                 855                 860

Leu Thr His Ala Lys Thr Phe Leu Arg Thr Leu Val Arg Gly Val Pro
865                 870                 875                 880

Glu Tyr Gly Cys Val Val Asn Leu Arg Lys Thr Val Val Asn Phe Pro
                885                 890                 895

Val Glu Asp Glu Ala Leu Gly Gly Thr Ala Phe Val Gln Met Pro Ala
            900                 905                 910

His Gly Leu Phe Pro Trp Cys Gly Leu Leu Leu Asp Thr Arg Thr Leu
        915                 920                 925

Glu Val Gln Ser Asp Tyr Ser Ser Tyr Ala Arg Thr Ser Ile Arg Ala
    930                 935                 940

Ser Leu Thr Phe Asn Arg Gly Phe Lys Ala Gly Arg Asn Met Arg Arg
945                 950                 955                 960

Lys Leu Phe Gly Val Leu Arg Leu Lys Cys His Ser Leu Phe Leu Asp
                965                 970                 975

Leu Gln Val Asn Ser Leu Gln Thr Val Cys Thr Asn Ile Tyr Lys Ile
            980                 985                 990

Leu Leu Leu Gln Ala Tyr Arg Phe  His Ala Cys Val Leu  Gln Leu Pro
        995                 1000                 1005

Phe His  Gln Gln Val Trp Lys  Asn Pro Thr Phe Phe  Leu Arg Val
    1010                 1015                 1020

Ile Ser  Asp Thr Ala Ser Leu  Cys Tyr Ser Ile Leu  Lys Ala Lys
    1025                 1030                 1035

Asn Ala  Gly Met Ser Leu Gly  Ala Lys Gly Ala Ala  Gly Pro Leu
    1040                 1045                 1050

Pro Ser  Glu Ala Val Gln Trp  Leu Cys His Gln Ala  Phe Leu Leu
    1055                 1060                 1065

Lys Leu  Thr Arg His Arg Val  Thr Tyr Val Pro Leu  Leu Gly Ser
    1070                 1075                 1080

Leu Arg  Thr Ala Gln Thr Gln  Leu Ser Arg Lys Leu  Pro Gly Thr
    1085                 1090                 1095

Thr Leu  Thr Ala Leu Glu Ala  Ala Ala Asn Pro Ala  Leu Pro Ser
    1100                 1105                 1110

Asp Phe  Lys Thr Ile Leu Asp

1115                1120


<210>  4
<211>  12
<212>  PRT
<213>  Homo sapiens

<400>  4

Ala Arg Pro Leu Glu His Gly Ser Asp Lys Ala Thr
1               5                   10


<210>  5
<211>  28
<212>  DNA
<213>  homo sapiens

<400>  5
taattaccat gggaatgccg cgcgctcc                                          28


<210>  6
<211>  18
<212>  DNA
<213>  homo sapiens

<400>  6
tcggtccacg cgtcctgc                                                     18


<210>  7
<211>  10
<212>  DNA
<213>  homo sapiens

<400>  7
gatccagagt                                                              10


<210>  8
<211>  10
<212>  DNA
<213>  homo sapiens

<400>  8
ctagactctg                                                              10